(19) Europäisches Patentamt

European Patent Office

Office européen des brevets

(11) **EP 4 736 893 A1**

(12) **EUROPEAN PATENT APPLICATION**

published in accordance with Art. 153(4) EPC

(43) Date of publication:
06.05.2026  Bulletin 2026/19

(21) Application number: 24831569.9

(22) Date of filing: 03.06.2024

(51) International Patent Classification (IPC):
*A61L 9/01* [(2006.01)]    *C02F 1/461* [(2023.01)]
*F24F 8/24* [(2021.01)]

(52) Cooperative Patent Classification (CPC):
A61L 9/01; C02F 1/461; F24F 8/24

(86) International application number:
PCT/JP2024/020195

(87) International publication number:
WO 2025/004699 (02.01.2025 Gazette 2025/01)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority:  30.06.2023  JP 2023108561

(71) Applicant: Panasonic Intellectual Property Management Co., Ltd.
Kadoma-shi, Osaka 571-0057 (JP)

(72) Inventors:
• YAMAGUCHI, Shotaro
  kadoma-shi, Osaka 571-0057 (JP)

• HAYASHI, Tomohiro
  kadoma-shi, Osaka 571-0057 (JP)
• IIDA, Kanako
  kadoma-shi, Osaka 571-0057 (JP)
• WADA, Takuya
  kadoma-shi, Osaka 571-0057 (JP)
• SHIMOOKA. Yusuke
  kadoma-shi, Osaka 571-0057 (JP)

(74) Representative: Grünecker Patent- und Rechtsanwälte
PartG mbB
Leopoldstraße 4
80802 München (DE)

(54)  **SPACE PURIFICATION DEVICE**

(57)  Space purification device (1) includes: electrolysis tank (10) configured to store first aqueous solution (L1); supply tank (20) configured to store second aqueous solution (L2) and supply chloride ions to first aqueous solution (L1); electrolysis tank-side anode (11) and electrolysis tank-side cathode (12) each provided in electrolysis tank (10); supply tank-side cathode (21) provided in supply tank (20); anion exchange membrane (30) disposed between electrolysis tank (10) and supply tank (20) and capable of permeating anions based on a voltage applied between electrolysis tank-side anode (11) and supply tank-side cathode (21); and current controller (40) configured to control the current to supplement chloride ions contained in first aqueous solution (L1) and decreased in number through the electrolysis, allowing chloride ions contained in second aqueous solution (L2) to permeate through anion exchange membrane (30) and be supplied to first aqueous solution (L1).

FIG. 1

**Description**

TECHNICAL FIELD

The present disclosure relates to a space purification device.

BACKGROUND ART

PTL 1 discloses an air purification device that removes bacteria, fungi, viruses, odors, and the like contained in the air using hypochlorous acid generated by electrolyzing an aqueous solution containing chloride ions.

Citation List

Patent Literature

PTL 1: Unexamined Japanese Patent Publication No. 2019-174032

SUMMARY OF THE INVENTION

When a conventional space purification device is downsized, the tank to store an aqueous solution used for electrolysis is also downsized. When the tank is downsized, the amount of the aqueous solution that can be stored is reduced as compared with the conventional space purification device. Therefore, there is a problem that when electrolysis is repeatedly performed in the downsized space purification device, the chloride ion concentration in the aqueous solution is likely to decrease, and the generation amount of hypochlorous acid is not stable.

The present disclosure has been made in view of the above problem, and an object thereof is to provide a space purification device capable of stably generating a desired amount of hypochlorous acid gas without externally supplying an aqueous solution containing chloride ions for a long period of time.

The space purification device according to the present disclosure includes: an electrolysis tank configured to store a first aqueous solution containing chloride ions; a supply tank configured to store a second aqueous solution containing chloride ions at a higher concentration than the first aqueous solution and supply chloride ions to the first aqueous solution; an electrolysis tank-side anode and an electrolysis tank-side cathode each provided in the electrolysis tank; a supply tank-side cathode provided in the supply tank; an anion exchange membrane disposed between the electrolysis tank and the supply tank and capable of permeating anions based on a voltage applied between the electrolysis tank-side anode and the supply tank-side cathode; a non-barrier membrane electrolysis part provided in the electrolysis tank and subjecting the first aqueous solution to non-barrier membrane electrolysis to generate hypochlorous acid with a first current flowing between the electrolysis tank-side anode and the electrolysis tank-side cathode; a barrier membrane electrolysis part provided over the electrolysis tank and the supply tank and performing barrier membrane electrolysis via the anion exchange membrane with a second current flowing between the electrolysis tank-side anode and the supply tank-side cathode; and a current controller configured to control the second current to supplement chloride ions contained in the first aqueous solution and decreased in number through the non-barrier membrane electrolysis, allowing chloride ions contained in the second aqueous solution to permeate through the anion exchange membrane and be supplied to the first aqueous solution.

According to the present disclosure, it is possible to provide a space purification device capable of stably generating a desired amount of hypochlorous acid gas without externally supplying an aqueous solution containing chloride ions for a long period of time.

BRIEF DESCRIPTION OF THE DRAWINGS

Fig. 1 is a perspective view illustrating a space purification device according to a first exemplary embodiment.
Fig. 2 is a front part cross-sectional view illustrating a space purification device according to a first exemplary embodiment.
Fig. 3 is a block diagram showing a current controller according to a first exemplary embodiment.
Fig. 4 is a front cross-sectional view illustrating a first modification of an electrolysis tank and a supply tank included in a space purification device according to a first exemplary embodiment.
Fig. 5 is a front cross-sectional view illustrating a second modification of an electrolysis tank and a supply tank included in a space purification device according to a first exemplary embodiment.

Fig. 6 is a schematic perspective view illustrating a space purification device according to a second exemplary embodiment.

Fig. 7 is a schematic plan view illustrating a space purification device according to a second exemplary embodiment.

DESCRIPTION OF EMBODIMENT

Hereinafter, specific exemplary embodiments of the present disclosure will be described in detail with reference to the drawings.

The xyz coordinates in the right-handed system shown in the drawings are for the convenience of explaining the positional relationship of the components. Unless otherwise noted, the z-axis plus direction is vertically upward. The xy plane is the horizontal plane, which is common to the drawings.

<First exemplary embodiment>

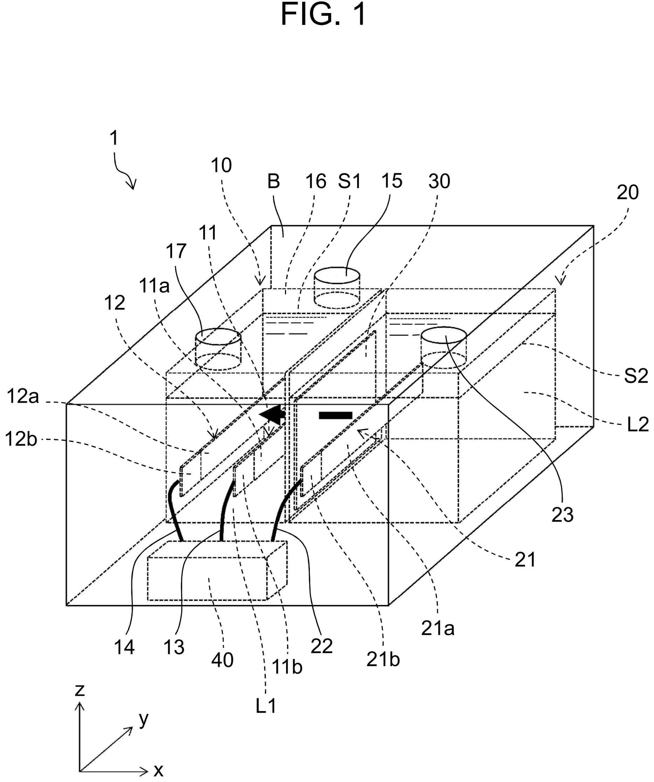

Fig. 1 is a perspective view illustrating the outline of space purification device 1 according to the first exemplary embodiment. In space purification device 1, first aqueous solution L1 containing chloride ions is electrolyzed in electrolysis tank 10 described later, and hypochlorous acid is generated and volatilized. In space purification device 1, the volatilized hypochlorous acid flows out to the outside space of case B constituting space purification device 1 to remove bacteria, fungi, viruses, odors, and the like contained in the air in the outside space of space purification device 1.

Space purification device 1 is installed in a room. The installation place of space purification device 1 is preferably a place where an air flow can occur. More specific examples of the installation place of space purification device 1 include the inside of a so-called air conditioner, which is an air-conditioning machine, the vicinity of an electric fan, the vicinity of a circulator, the vicinity of a ceiling fan, the inside of a humidification device, the inside of an air purifier, and the top of a desk.

Space purification device 1 includes case B, electrolysis tank 10, supply tank 20, anion exchange membrane 30, and current controller 40.

Case B houses electrolysis tank 10, supply tank 20, anion exchange membrane 30, and current controller 40. That is, space purification device 1 may be a unit integrated by case B. The shape of case B can be appropriately changed depending on the place where space purification device 1 is installed, and may be, for example, a rectangular parallelepiped shape or a cylindrical shape. The size of space purification device 1 is, for example, so small that it can be housed inside an air conditioner, and is, for example, about 10 cm $\times$ 7 cm $\times$ 4 cm.

Electrolysis tank 10 is a tank configured to store first aqueous solution L1 containing chloride ions. Electrolysis tank 10 has, for example, a box shape. Fig. 1 illustrates a state in which first aqueous solution L1 is stored in electrolysis tank 10. First aqueous solution L1 is, for example, a dilute sodium chloride aqueous solution or a dilute potassium chloride aqueous solution having a predetermined chloride ion concentration.

The "predetermined chloride ion concentration" of first aqueous solution L1 includes both a chloride ion concentration having a predetermined numerical range and a chloride ion concentration having a predetermined numerical value. More specifically, the chloride ion concentration of first aqueous solution L1 may be, for example, 1 g/L to 50 g/L, or may be 10 g/L. In other words, for example, the mass percent concentration of the dilute sodium chloride aqueous solution or the dilute potassium chloride aqueous solution may be 0.1% to 5%, or may be 1%. When the predetermined chloride ion concentration is the numerical range or the numerical value, it is possible to generate hypochlorous acid, which is necessary for spatial purification, and inhibit the generation of chlorine, which may be generated at the same time.

Supply tank 20 is a tank configured to store second aqueous solution L2 containing chloride ions. Chloride ions contained in second aqueous solution L2 in supply tank 20 permeate through anion exchange membrane 30 and are supplied to first aqueous solution L1 in electrolysis tank 10.

Supply tank 20 is a tank configured to store second aqueous solution L2 containing chloride ions, and supply chloride ions to first aqueous solution L1. Fig. 1 illustrates a state in which second aqueous solution L2 is stored in supply tank 20. The chloride ion concentration of second aqueous solution L2 is higher than the chloride ion concentration of first aqueous solution L1. Second aqueous solution L2 is, for example, a saturated sodium chloride aqueous solution, a high concentration sodium chloride aqueous solution, a saturated potassium chloride aqueous solution, a high concentration potassium chloride aqueous solution, or a high concentration hydrochloric acid. More specifically, second aqueous solution L2 is, for example, a 10% to 27% sodium chloride aqueous solution, a 10% to 29% potassium chloride aqueous solution, or a 10% to 25% hydrochloric acid. Second aqueous solution L2 may be in a state where sodium chloride or potassium chloride is separated and precipitated on the bottom of supply tank 20.

Assuming continuous use for 8 hours every day for 1 year, the volumes of electrolysis tank 10 and the supply tank 20 are preferably determined, for example, such that the volume of supply tank 20 is about 12 times or more the volume of electrolysis tank 10. With such a volume ratio, supply tank 20 can store a sufficient amount of second aqueous solution L2 containing chloride ions, which needs to be supplied to first aqueous solution L1 in electrolysis tank 10. Therefore, supply tank 20 can stably supply chloride ions from second aqueous solution L2 stored in supply tank 20 to first aqueous solution L1 stored in electrolysis tank 10. The amount of first aqueous solution L1 stored in electrolysis tank 10 is, for example, about 2 to 10 mL.

Anion exchange membrane 30 is a membrane member disposed between electrolysis tank 10 and supply tank 20 and capable of permeating anions based on a voltage applied between electrolysis tank 10 and supply tank 20. More specifically, when a voltage is applied between the electrolysis tank-side anode plate (electrolysis tank-side anode 11 in the first exemplary embodiment) and the supply tank-side cathode plate (supply tank-side cathode 21 in the first exemplary embodiment), each of which is described later, barrier membrane electrolysis is performed via anion exchange membrane 30. Through the barrier membrane electrolysis using the electrolysis tank-side anode plate and the supply tank-side cathode plate, chloride ions contained in second aqueous solution L2 permeate through anion exchange membrane 30 and are supplied to first aqueous solution L1 (shown in a black thick arrow directed in the negative direction of the x-axis in Fig. 1).

In the exemplary embodiment, anion exchange membrane 30 is not a type of anion exchange membrane that permeates anions not with electricity but with osmotic pressure. The anion exchange membrane 30 does not permeate cations, sodium ions. More specifically, when chloride ions contained in second aqueous solution L2 permeate through anion exchange membrane 30 and are supplied to first aqueous solution L1 through the barrier membrane electrolysis using the electrolysis tank-side anode plate and the supply tank-side cathode plate, cations, sodium ions, do not permeate through anion exchange membrane 30. Anion exchange membrane 30 is, for example, a hydrocarbon-based anion exchange membrane. Examples thereof include membranes having characteristics of monovalent anion selective permeability, alkali resistance, and high temperature resistance.

Anion exchange membrane 30 is disposed between electrolysis tank 10 and supply tank 20. For example, the surface of electrolysis tank 10 facing supply tank 20 (the yz plane on the positive side of the x-axis) and the surface of supply tank 20 facing electrolysis tank 10 (the yz plane on the negative side of the x-axis) may be formed of a frame member, respectively. When each of the surfaces where electrolysis tank 10 and supply tank 20 face each other is formed of a frame member, anion exchange membrane 30 may be disposed such that it is fitted into the frame members.

Current controller 40 controls the currents used for the non-barrier membrane electrolysis and the barrier membrane electrolysis. More specifically, current controller 40 controls the current used for the non-barrier membrane electrolysis, which is performed by using a pair of electrolysis tank-side anode 11 and electrolysis tank-side cathode 12 each disposed in electrolysis tank 10. In addition, current controller 40 controls the current used for the barrier membrane electrolysis, which is performed via anion exchange membrane 30 by using a pair of electrolysis tank-side anode 11 and supply tank-side cathode 21 over electrolysis tank 10 and supply tank 20. Electrolysis tank-side anode 11 is used for both the non-barrier membrane electrolysis and the barrier membrane electrolysis. That is, space purification device 1 according to the exemplary embodiment has one anode and two cathodes, three electrodes in total. Since supply tank 20 has only a cathode, chlorine is not generated in supply tank 20 by the chemical reactions described later.

Hereinafter, details of each configuration will be described more specifically with reference to Figs. 1 to 3.

As illustrated in Fig. 1, electrolysis tank 10 includes electrolysis tank-side anode 11, electrolysis tank-side cathode 12, wiring 13, wiring 14, inflow port 15, mixing space 16, and outflow port 17.

Electrolysis tank-side anode 11 and electrolysis tank-side cathode 12 are a pair of electrodes to be used for the electrolysis of first aqueous solution L1. As illustrated in Fig. 1, no membrane such as an ion exchange membrane is disposed between electrolysis tank-side anode 11 and electrolysis tank-side cathode 12. That is, the electrolysis of first aqueous solution L1 performed using a pair of electrolysis tank-side anode 11 and electrolysis tank-side cathode 12 is non-barrier membrane electrolysis. Through the non-barrier membrane electrolysis of first aqueous solution L1 performed using a pair of electrolysis tank-side anode 11 and electrolysis tank-side cathode 12, hypochlorous acid to be used for space purification is generated.

Each of electrolysis tank-side anode 11 and electrolysis tank-side cathode 12 has a plate shape. That is, electrolysis tank-side anode 11 is an electrolysis tank-side anode plate having a plate shape, and electrolysis tank-side cathode 12 is an electrolysis tank-side cathode plate having a plate shape. The plate shape includes a rectangular shape. Hereinafter, electrolysis tank-side anode 11 is also referred to as electrolysis tank-side anode plate. Also, electrolysis tank-side cathode 12 is also referred to as electrolysis tank-side cathode plate.

For example, the case where the plate shapes of the electrolysis tank-side anode plate and the electrolysis tank-side cathode plate are rectangular will be described. The electrolysis tank-side anode plate and the electrolysis tank-side cathode plate are disposed such that the short direction of the rectangle is along the vertical direction (z-axis direction). With the disposition, it is possible to prevent a case where bubbles generated by chemical reaction adhere to both surfaces of the rectangle of each electrode. Further, as compared with the case where the long direction of the rectangle is disposed along the vertical direction (z-axis direction), it is possible to prevent a case where bubbles generated at the lower part (on the negative side of z-axis) of the electrolysis tank-side anode plate and the electrolysis tank-side cathode plate adhere to the upper part (on the positive side of z-axis) of the electrolysis tank-side anode plate and the electrolysis tank-side cathode plate when the short direction is disposed along the vertical direction (z-axis direction).

The electrolysis tank-side anode plate and the electrolysis tank-side cathode plate are disposed such that the long direction of the rectangle is along the horizontal direction (y-axis direction). In other words, the electrolysis tank-side anode plate and the electrolysis tank-side cathode plate are disposed such that the planes (yz planes) of the rectangles thereof face each other at a predetermined interval. The predetermined interval is an interval suitable for the electrolysis performed using a pair of the electrolysis tank-side anode plate and the electrolysis tank-side cathode plate.

The electrolysis tank-side anode plate includes electrolysis tank-side anode plate immersion part 11a and electrolysis tank-side anode plate protrusion part 11b. Similarly, the electrolysis tank-side cathode plate includes electrolysis tank-side cathode plate immersion part 12a and electrolysis tank-side cathode plate protrusion part 12b.

The electrolysis tank-side anode plate and the electrolysis tank-side cathode plate are inserted from the outside of electrolysis tank 10 to the inside thereof. In Fig. 1, for example, the electrolysis tank-side anode plate and the electrolysis tank-side cathode plate are inserted in the horizontal direction (y-axis direction) from the lateral side (xz plane side on the negative side of y-axis) of electrolysis tank 10. Electrolysis tank-side anode plate immersion part 11a and electrolysis tank-side cathode plate immersion part 12a inserted into electrolysis tank 10 are disposed inside electrolysis tank 10, and entirely immersed in first aqueous solution L1. In other words, first aqueous solution L1 is stored in electrolysis tank 10 such that electrolysis tank-side anode plate immersion part 11a and electrolysis tank-side cathode plate immersion part 12a are entirely immersed therein. That is, first aqueous solution L1 is stored in electrolysis tank 10 such that liquid level S1 of first aqueous solution L1 is higher than the upper ends (ends on the positive side of z-axis) of electrolysis tank-side anode plate immersion part 11a and electrolysis tank-side cathode plate immersion part 12a.

Electrolysis tank-side anode plate protrusion part 11b and electrolysis tank-side cathode plate protrusion part 12b are disposed outside electrolysis tank 10. Wiring 13 and wiring 14 are electric wires through which current flows. Electrolysis tank-side anode plate protrusion part 11b and electrolysis tank-side cathode plate protrusion part 12b are electrically connected to current controller 40 via wiring 13 and wiring 14, respectively.

As the electrolysis tank-side anode plate and the electrolysis tank-side cathode plate, for example, a platinum iridium titanium electrode, a platinum electrode, a ruthenium titanium electrode, or an iridium titanium oxide electrode may be used.

Inflow port 15 is an opening into which air in the outside space of case B flows. That is, inflow port 15 is an opening into which air in the outside space of space purification device 1 flows. In Fig. 1, for example, inflow port 15 is provided on the upper surface (the xy plane on the positive side of z-axis) of electrolysis tank 10, but inflow port 15 may be disposed above liquid level S1 of first aqueous solution L1. For example, the shape of inflow port 15 may be a cylindrical shape as illustrated in Fig. 1, or may be a polygonal tube shape.

Mixing space 16 is a space formed on the upper side (on the positive side of z-axis) of electrolysis tank 10 in a state where first aqueous solution L1 is stored in electrolysis tank 10. Mixing space 16 is a space in which the hypochlorous acid generated by the non-barrier membrane electrolysis of first aqueous solution L1 performed using a pair of the electrolysis tank-side anode plate and the electrolysis tank-side cathode plate is mixed with the air in the outside space having flowed in from inflow port 15. The hypochlorous acid generated by the non-barrier membrane electrolysis includes hypochlorous acid gas volatilized and gasified, and hypochlorous acid dissolved in first aqueous solution L1. The hypochlorous acid gas is contained in the air having flowed in from inflow port 15, and flows out to the outside space from outflow port 17 described later. The hypochlorous acid dissolved in first aqueous solution L1 comes into gas-liquid contact with the air having flowed in from inflow port 15 to flow out to the outside space from outflow port 17 described later.

Outflow port 17 is an opening to flow out a mixed air to the outside space of case B, the air having flowed in from inflow port 15 and hypochlorous acid generated by the non-barrier membrane electrolysis of first aqueous solution L1 having been mixed in the mixed air. That is, outflow port 17 is an opening through which the mixed air flows out to the outside space of space purification device 1. In Fig. 1, as in inflow port 15, for example, outflow port 17 is provided on the upper surface (the xy plane on the positive side of z-axis) of electrolysis tank 10, but outflow port 17 may be disposed above liquid level S1 of first aqueous solution L1. The shape of outflow port 17 is the same as that of inflow port 15, and for example, may be a cylindrical shape as illustrated in Fig. 1, or may be a polygonal tube shape.

At least one of inflow port 15 and outflow port 17 may have an openable or detachable lid (not illustrated). The lid may be configured to be closed when space purification device 1 is transported or moved, and opened or detached when space purification device 1 is used. Although inflow port 15 and outflow port 17 are described as different configurations, inflow port 15 and outflow port 17 may serve as both an inflow port and an outflow port, respectively, depending on the direction of the wind flowing into space purification device 1.

The air containing hypochlorous acid flowing out from outflow port 17 to the outside space of space purification device 1 purifies the outside space. That is, the air containing hypochlorous acid removes bacteria, fungi, viruses, odors, and the like contained in the air in the outside space of case B.

Supply tank 20 includes supply tank-side cathode 21, wiring 22, and discharge port 23. Supply tank-side cathode 21 is an electrode that is paired with electrolysis tank-side anode 11 and used for the electrolysis of second aqueous solution L2. As illustrated in Fig. 1, anion exchange membrane 30 is disposed between electrolysis tank-side anode 11 and supply tank-side cathode 21. That is, the electrolysis of second aqueous solution L2 performed using a pair of electrolysis tank-side anode 11 and supply tank-side cathode 21 is barrier membrane electrolysis. In short, electrolysis tank-side anode 11 is used for both the non-barrier membrane electrolysis and the barrier membrane electrolysis. Through the barrier membrane electrolysis of second aqueous solution L2 performed using a pair of electrolysis tank-side anode 11 and supply tank-side cathode 21, chloride ions are supplied from second aqueous solution L2 to first aqueous solution L1.

Supply tank-side cathode 21 is a supply tank-side cathode plate having a plate shape. The plate shape includes a rectangular shape. Hereinafter, supply tank-side cathode 21 is also referred to as supply tank-side cathode plate.

For example, the case where the plate shape of the supply tank-side cathode plate is also rectangular similarly to that of the electrolysis tank-side anode plate will be described. As illustrated in Fig. 1, the supply tank-side cathode plate is disposed such that the short direction of the rectangle is along the vertical direction (z-axis direction). The supply tank-side cathode plate is disposed such that the long direction of the rectangle is along the horizontal direction (y-axis direction).

The electrolysis tank-side anode plate and the supply tank-side cathode plate are close to anion exchange membrane 30, respectively. The "close" in the present specification includes both: a state in which the electrolysis tank-side anode plate and the supply tank-side cathode plate are close to anion exchange membrane 30 at a predetermined interval; and a state in which the electrolysis tank-side anode plate and the supply tank-side cathode plate are in contact with anion exchange membrane 30.

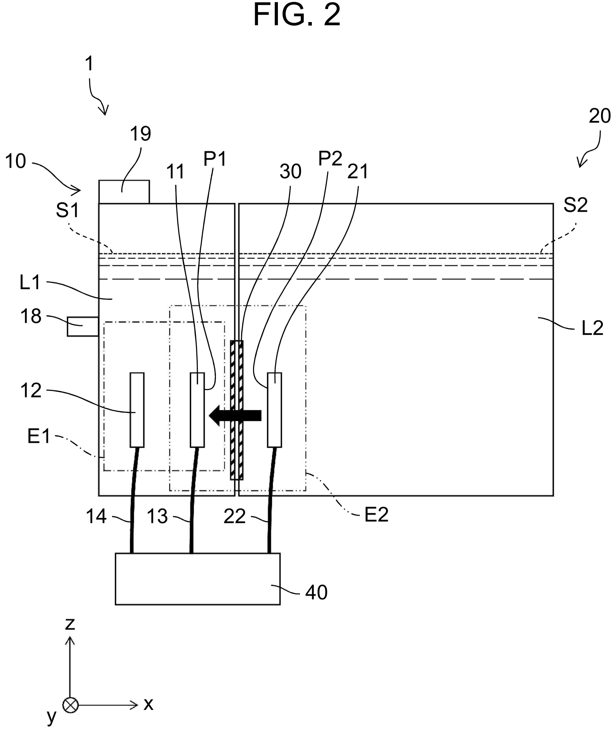

As illustrated in Fig. 2, the rectangular plane on the side of anion exchange membrane 30 (yz plane on the positive side of the x-axis) of the electrolysis tank-side anode plate having a plate shape is defined as plane P1. The rectangular plane on the side of anion exchange membrane 30 (yz plane on the negative side of the x-axis) of the supply tank-side cathode plate having a plate shape is defined as plane P2. Plane P1 and plane P2 are disposed to face each other via anion exchange membrane 30. With the disposition, an electric field can be uniformly generated between the electrolysis tank-side anode plate and the supply tank-side cathode plate.

The electrolysis tank-side anode plate is disposed between the electrolysis tank-side cathode plate and anion exchange membrane 30. With the disposition, the potential difference between the electrolysis tank-side anode plate and the electrolysis tank-side cathode plate; and the potential difference between the electrolysis tank-side anode plate and the supply tank-side cathode plate can be kept small.

Supply tank-side cathode plate includes supply tank-side cathode plate immersion part 21a and supply tank-side cathode plate protrusion part 21b. The supply tank-side cathode plate is inserted from the outside of supply tank 20 to the inside thereof. In Fig. 1, for example, the supply tank-side cathode plate is inserted in the horizontal direction (y-axis direction) from the lateral side (xz plane side on the negative side of y-axis) of supply tank 20. Supply tank-side cathode plate immersion part 21a inserted into supply tank 20 is disposed inside supply tank 20, and entirely immersed in second aqueous solution L2. In other words, second aqueous solution L2 is stored in supply tank 20 such that supply tank-side cathode plate immersion part 21a is entirely immersed therein. That is, second aqueous solution L2 is stored in supply tank 20 such that liquid level S2 of second aqueous solution L2 is higher than the upper end (end on the positive side of z-axis) of supply tank-side cathode plate immersion part 21a.

As illustrated in Fig. 1, supply tank-side cathode plate protrusion part 21b is disposed outside supply tank 20. Wiring 22 is an electric wire through which current flows. Supply tank-side cathode plate protrusion part 21b is electrically connected to current controller 40 via wiring 22.

As the supply tank-side cathode plate, for example, a platinum iridium titanium electrode, a platinum electrode, a ruthenium titanium electrode, or an iridium titanium oxide electrode may be used.

Discharge port 23 is an opening to discharge hydrogen gas generated by the barrier membrane electrolysis of second aqueous solution L2 to the outside space of case B. Discharge port 23 may be, for example, a check valve. When a check valve is used as discharge port 23, the hydrogen gas in supply tank 20 is discharged to the outside space, but the inflow of the gas such as air from the outside space can be inhibited. When the barrier membrane electrolysis of second aqueous solution L2 is repeated, hydrogen gas accumulates in supply tank 20, and the internal pressure of supply tank 20 increases. The check valve is opened by the pressure, and the hydrogen gas is discharged to the outside space of supply tank 20.

Fig. 2 is a front part cross-sectional view illustrating space purification device 1 of Fig. 1. In Fig. 2, case B illustrated in Fig. 1 is omitted. As illustrated in Fig. 2, space purification device 1 may further include water level detection unit 18 and water supply unit 19. Water level detection unit 18 detects the position of liquid level S1 of first aqueous solution L1. Water level detection unit 18 is, for example, a water level sensor. Water level detection unit 18 is at least disposed above (on the positive side of z-axis) the upper ends (ends on the positive side of z-axis) of electrolysis tank-side anode plate immersion part 11a, electrolysis tank-side cathode plate immersion part 12a, and supply tank-side cathode plate immersion part 21a.

Water supply unit 19 supplies water to electrolysis tank 10 based on the position of liquid level S1 detected by water level detection unit 18. More specifically, water supply unit 19 supplies water to electrolysis tank 10 so that liquid level S1 is not lower than the upper ends (ends on the positive side of z-axis) of electrolysis tank-side anode plate immersion part 11a, electrolysis tank-side cathode plate immersion part 12a, and supply tank-side cathode plate immersion part 21a. Water supply unit 19 may be, for example, a Peltier element capable of cooling and condensing moisture contained in the air to form water droplets, or a water tank capable of storing water. Water supply unit 19 is disposed at a position where water can be supplied to electrolysis tank 10, and may be disposed at the upper side of electrolysis tank 10, or may be disposed at the lateral side or the bottom side of electrolysis tank 10.

When space purification device 1 includes water level detection unit 18 and water supply unit 19, electrolysis tank-side anode plate immersion part 11a and electrolysis tank-side cathode plate immersion part 12a can be kept immersed in first aqueous solution L1. Therefore, it is possible to inhibit electrolysis tank-side anode plate immersion part 11a and electrolysis tank-side cathode plate immersion part 12a being exposed to the air due to the decrease in the amount of first aqueous solution L1, and maintain the electrolysis efficiency of the non-barrier membrane electrolysis.

As illustrated in Fig. 2, space purification device 1 according to the exemplary embodiment includes non-barrier membrane electrolysis part E1 and barrier membrane electrolysis part E2. Current controller 40 can control the first current flowing in non-barrier membrane electrolysis part E1 and the second current flowing in barrier membrane electrolysis part E2 so that the chemical reaction occurring in non-barrier membrane electrolysis part E1 and the chemical reaction occurring in barrier membrane electrolysis part E2 are controlled.

Non-barrier membrane electrolysis part E1 is provided in electrolysis tank 10. In non-barrier membrane electrolysis part E1, the first current flows between electrolysis tank-side anode 11 and electrolysis tank-side cathode 12 to subject first aqueous solution L1 to non-barrier membrane electrolysis, thereby generating hypochlorous acid. In other words, non-barrier membrane electrolysis part E1 includes electrolysis tank-side anode 11 and electrolysis tank-side cathode 12.

The barrier membrane electrolysis part E2 is provided over electrolysis tank 10 and supply tank 20. The second current flows between electrolysis tank-side anode 11 and supply tank-side cathode 21 to perform barrier membrane electrolysis via anion exchange membrane 30. In other words, barrier membrane electrolysis part E2 includes electrolysis tank-side anode 11, supply tank-side cathode 21, and anion exchange membrane 30.

Here, the chemical reaction occurring in non-barrier membrane electrolysis part E1 provided in electrolysis tank 10 and the chemical reaction occurring in barrier membrane electrolysis part E2 provided over electrolysis tank 10 and supply tank 20 will be described in detail. Hereinafter, a case where first aqueous solution L1 and second aqueous solution L2 each containing chloride ions are sodium chloride aqueous solutions will be described.

[Non-barrier membrane electrolysis part E1 (electrolysis tank 10)]

Sodium chloride (NaCl) contained in a sodium chloride aqueous solution is ionized into Na+ and Cl- in water. When a predetermined voltage is applied to non-barrier membrane electrolysis part E1, current flows between electrolysis tank-side anode 11 and supply tank-side cathode 21 and electrons move, and thereby the following chemical reactions occur.

· Reaction formula (a): electrolysis tank-side anode 11 (chlorine is generated)

[Chemical formula 1]          $2Cl^- \rightarrow Cl_2 + 2e^-$

· Reaction formula (b): electrolysis tank-side cathode 12 (hydrogen is generated)

[Chemical formula 2]

$$H_2O + e^- \rightarrow \frac{1}{2}H_2 + OH^-$$

· Reaction formula (c): electrolysis tank-side anode 11 (oxygen is generated)

[Chemical formula 3]

$$OH^- \rightarrow \frac{1}{2}H_2O + \frac{1}{4}O_2 + e^-$$

· Reaction formula (d): in first aqueous solution L1 (hypochlorous acid is generated)

[Chemical formula 4]          $Cl_2 + H_2O \leftrightarrows HCl + HClO$

· Reaction formula (e): hypochlorous acid generation reaction in electrolysis tank-side anode 11 (formula (a) + formula (d))

[Chemical formula 5]

$$Cl^- + \frac{1}{2}H_2O \rightarrow \frac{1}{2}HCl + \frac{1}{2}HClO + e^-$$

· Reaction formula (f): anion exchange membrane 30
When a voltage is applied to non-barrier membrane electrolysis part E1 and current flows, and first aqueous solution L1 stored in electrolysis tank 10 loses one electron (e-), a chloride ion Cl- permeates through anion exchange membrane 30 from second aqueous solution L2 stored in supply tank 20 and is supplied to first aqueous solution L1 in electrolysis tank 10.

[Chemical formula 6]     $e^- \rightarrow Cl^-$

Here, the ratio of the current used for the hypochlorous acid generation reaction of the reaction formula (e) is defined as "x", and the ratio of the current used for the oxygen generation reaction of the reaction formula (c) is defined as "1-x". The ratio of the current used for the reaction formula (b) of the non-barrier membrane electrolysis is defined as "y", and the ratio of the current used for the reaction formula (f) of the barrier membrane electrolysis is defined as "1-y". When the above x and y are applied to the reaction formula (b) + (c) + (e) + (f), the following reaction formula (g) is obtained.

· Reaction formula (g): reaction formula (b) + (c) + (e) + (f)

[Chemical formula 7]

$$x\left(Cl^- + \frac{1}{2}H_2O\right) + (1-x)OH^- + y(H_2O + e^-) + (1-y)e^-$$

$$\rightarrow x\left(\frac{1}{2}HCl + \frac{1}{2}HClO + e^-\right) + (1-x)\left(\frac{1}{2}H_2O + \frac{1}{4}O_2 + e^-\right)$$

$$+y\left(\frac{1}{2}H_2 + OH^-\right) + (1-y)Cl^-$$

When the non-barrier membrane electrolysis is performed in non-barrier membrane electrolysis part E1, chloride ions are consumed and thus decreased in electrolysis tank 10. However, in space purification device 1 according to the exemplary embodiment, chloride ions are supplied from second aqueous solution L2 stored in supply tank 20 to first aqueous solution L1 stored in electrolysis tank 10.

Here, the following is the condition where chloride ions (Cl-) in an amount consumed by the non-barrier membrane electrolysis are supplied from second aqueous solution L2 stored in supply tank 20 to first aqueous solution L1 stored in electrolysis tank 10, and Cl- is not apparently increased or decreased in electrolysis tank 10. In the reaction, hypochlorous acid (HClO) volatilizes as a gas, and therefore is not included in the following formulas.

· Reaction formula (h): condition where Cl- is not apparently increased or decreased

[Chemical formula 8]

$$x(Cl^-) = x\left(\frac{1}{2}Cl^-\right) + (1-y)Cl^-$$

After deformation, the following reaction formula (i) is obtained.

· Reaction formula (i): deformation of reaction formula (h)

[Chemical formula 9]

$$y = 1 - \frac{x}{2}$$

When y is substituted into the above reaction formula (g), the following reaction formula (j) is obtained.

· Reaction formula (j): reaction formula (g) + (i)

[Chemical formula 10]

$$x\left(Cl^- + \frac{1}{2}H_2O\right) + (1-x)OH^- + \left(1 - \frac{x}{2}\right)(H_2O + e^-) + \left(1 - \left(1 - \frac{x}{2}\right)\right)e^-$$

$$\rightarrow x\left(\frac{1}{2}HCl + \frac{1}{2}HClO + e^-\right) + (1-x)\left(\frac{1}{2}H_2O + \frac{1}{4}O_2 + e^-\right)$$

$$+ \left(1 - \frac{x}{2}\right)\left(\frac{1}{2}H_2 + OH^-\right) + \left(1 - \left(1 - \frac{x}{2}\right)\right)Cl^-$$

After deformation, the following reaction formula (k) is obtained.

· Reaction formula (k): deformation of reaction formula (j)

[Chemical formula 11]

$$\frac{1}{2}H_2O \rightarrow \frac{x}{2}HClO + \left(\frac{1-x}{4}\right)O_2 + \frac{1}{2}\left(1 - \frac{x}{2}\right)H_2$$

From the above reaction formula (k), for example, when two electrons flow into non-barrier membrane electrolysis part E1, one hypochlorous acid (HClO) is generated. For example, when four electrons flow into non-barrier membrane electrolysis part E1, one oxygen is generated. For example, when two electrons flow into non-barrier membrane electrolysis part E1, one hydrogen is generated.

Since the acid dissociation constant of hypochlorous acid (HClO) is about 7.5, it is necessary that the pH of first aqueous solution L1 remains unchanged. In the above reaction formula (k), hydroxide ions and hydrogen ions, which are factors of pH change, react with each other to become water, and disappear in the reaction formula. Therefore, the increase or decrease in pH can be inhibited under the condition where chloride ions (Cl-) in an amount consumed by the non-barrier membrane electrolysis are supplied from second aqueous solution L2 stored in supply tank 20 to first aqueous solution L1 stored in electrolysis tank 10, and Cl- is not apparently increased or decreased in electrolysis tank 10.

As described above, when the number of electrons flowing into non-barrier membrane electrolysis part E1 changes, that is, when the ratio between the current flowing into non-barrier membrane electrolysis part E1 and the current flowing into barrier membrane electrolysis part E2 changes, the supply amount of chloride ions supplied from supply tank 20 to electrolysis tank 10 changes. For example, when the ratio of the current used for the non-barrier membrane electrolysis is larger than the ratio of the current under the condition where chloride ions (Cl-) are supplied from supply tank 20 and Cl- does not apparently increase or decrease in electrolysis tank 10 (the following reaction formula (1)), the supply amount of Cl- supplied from supply tank 20 to electrolysis tank 10 decreases and Cl- decreases in electrolysis tank 10.

· Reaction formula (1)

## [Chemical formula 12]

$$y > 1 - \frac{x}{2}$$

When Cl- decreases in electrolysis tank 10, the electrolysis efficiency of the non-barrier membrane electrolysis decreases. When the non-barrier membrane electrolysis is continued in a state where the electrolysis efficiency has decreased, Cl- gradually increases in electrolysis tank 10, and reaches the amount at which Cl- does not apparently increase or decrease in electrolysis tank 10.

On the other hand, when the ratio of the current used for the non-barrier membrane electrolysis is less than the raftio of the current under the condition where chloride ions (Cl-) are supplied from supply tank 20 and Cl- does not apparently increase or decrease in electrolysis tank 10 (the following reaction formula (m)), the supply amount of Cl- supplied from supply tank 20 to electrolysis tank 10 increases and Cl- increases in electrolysis tank 10.

· Reaction formula (m)

## [Chemical formula 13]

$$y < 1 - \frac{x}{2}$$

When Cl- increases in electrolysis tank 10, the electrolysis efficiency of the non-barrier membrane electrolysis increases. When the non-barrier membrane electrolysis is continued in a state where the electrolysis efficiency has increased, Cl- gradually decreases in electrolysis tank 10, and reaches the amount at which Cl- does not apparently increase or decrease in electrolysis tank 10.

In addition, when Cl- increases in electrolysis tank 10, the equilibrium of the reaction formula (d) shifts to the left, and the generation amount of chlorine increases. In other words, when the current flowing into non-barrier membrane electrolysis part E1 and the current flowing into barrier membrane electrolysis part E2 are adjusted so that Cl- does not apparently increase or decrease in electrolysis tank 10, hypochlorous acid can be generated while suppressing the generation of chlorine.

[Barrier membrane electrolysis part E2]

The reaction in supply tank 20 will be described. The electrode disposed in supply tank 20 is only supply tank-side cathode 21. When a predetermined voltage is applied to barrier membrane electrolysis part E2, current flows and electrons move, and thereby the following chemical reactions occur.

· Reaction formula (n): electrolysis tank-side cathode 12 (hydrogen is generated)

## [Chemical formula 14]

$$H_2O + e^- \rightarrow \frac{1}{2}H_2 + OH^-$$

· Reaction formula (o): anion exchange membrane 30
When a voltage is applied to barrier membrane electrolysis part E2 and current flows, a chloride ion (Cl-) contained in second aqueous solution L2 stored in supply tank 20 permeates through anion exchange membrane 30 and is supplied to first aqueous solution L1, and second aqueous solution L2 obtains one electron (e-).

[Chemical formula 15] $\quad Cl^- \rightarrow e^-$

As described above, the chemical reaction occurring in non-barrier membrane electrolysis part E1 provided in electrolysis tank 10 and the chemical reaction occurring in barrier membrane electrolysis part E2 provided over electrolysis tank 10 and supply tank 20 have been described in detail.

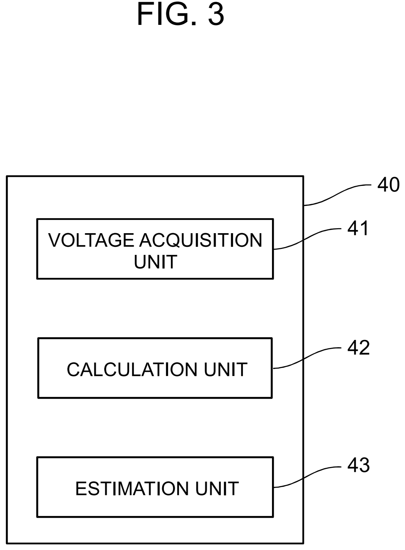

Current controller 40 controls the above chemical reactions. More specifically, current controller 40 controls the second current to supplement chloride ions contained in first aqueous solution L1 and decreased in number through the non-barrier membrane electrolysis in non-barrier membrane electrolysis part E1. Current controller 40 controls the second current, allowing chloride ions contained in second aqueous solution L2 to permeate through anion exchange membrane 30 and be supplied to first aqueous solution L1. Hereinafter, details will be described with reference to Fig. 3.

Fig. 3 is a block diagram showing current controller 40 according to the first exemplary embodiment. As illustrated in Fig. 3, current controller 40 includes voltage acquisition unit 41, calculation unit 42, and estimation unit 43.

Voltage acquisition unit 41 acquires the voltage between electrolysis tank-side anode 11 and electrolysis tank-side cathode 12. Voltage acquisition unit 41 is, for example, a voltmeter. Calculation unit 42 calculates the electric conductivity of first aqueous solution L1 based on the voltage acquired by voltage acquisition unit 41. Estimation unit 43 estimates the chloride ion concentration of first aqueous solution L1 based on the electric conductivity of first aqueous solution L1 calculated by calculation unit 42.

Current controller 40 controls the first current flowing in non-barrier membrane electrolysis part E1 illustrated in Fig. 2 and the second current flowing in barrier membrane electrolysis part E2 so that the chloride ion concentration of first aqueous solution L1 is maintained at a predetermined concentration. Hereinafter, three examples of current control by current controller 40 will be described.

[1. Case of simultaneously flowing first current and second current]

When current controller 40 simultaneously flows the first current and the second current, the controls (1) to (3) below are performed.

(1) When the chloride ion concentration of first aqueous solution L1 estimated by estimation unit 43 is lower than a predetermined concentration: the current ratio between the first current and the second current is changed to increase the amount of chloride ions permeating through anion exchange membrane 30 from second aqueous solution L2 and supplied to first aqueous solution L1. More specifically, the current ratio of the first current is decreased, and the current ratio of the second current is increased.

(2) When the chloride ion concentration of first aqueous solution L1 estimated by estimation unit 43 is higher than the predetermined concentration: the current ratio between the first current and the second current is changed to decrease the amount of chloride ions permeating through anion exchange membrane 30 from second aqueous solution L2 and supplied to first aqueous solution L1. More specifically, the current ratio of the first current is increased, and the current ratio of the second current is decreased.

(3) When the chloride ion concentration of first aqueous solution L1 estimated by estimation unit 43 is equal to the predetermined concentration: the current ratio between the first current and the second current is not changed.

[2. Case of flowing first current at predetermined value and simultaneously controlling second current]

When current controller 40 flows the first current at a predetermined value and simultaneously controls the second current, the controls (1) to (3) below are performed.

(1) When the chloride ion concentration of first aqueous solution L1 estimated by estimation unit 43 is lower than a predetermined concentration: the second current flows to increase the amount of chloride ions permeating through anion exchange membrane 30 from second aqueous solution L2 and supplied to first aqueous solution L1.

(2) When the chloride ion concentration of first aqueous solution L1 estimated by estimation unit 43 is higher than the predetermined concentration: the second current is stopped to stop supplying chloride ions from second aqueous solution L2 to first aqueous solution L1.

(3) When the chloride ion concentration of first aqueous solution L1 estimated by estimation unit 43 is equal to the predetermined concentration: the first current and the second current are not changed.

[3. Case of flowing first current or second current]

When current controller 40 flows the first current or the second current, the controls (1) to (3) below are performed.

(1) When the chloride ion concentration of first aqueous solution L1 estimated by estimation unit 43 is lower than a predetermined concentration: the first current is stopped and the second current simultaneously flows to increase the amount of chloride ions permeating through anion exchange membrane 30 from second aqueous solution L2 and supplied to first aqueous solution L1.

(2) When the chloride ion concentration of first aqueous solution L1 estimated by estimation unit 43 is higher than the predetermined concentration: the second current is stopped and the first current simultaneously flows to stop supplying chloride ions from second aqueous solution L2 to first aqueous solution L1.

(3) When the chloride ion concentration of first aqueous solution L1 estimated by estimation unit 43 is equal to the predetermined concentration: the first current and the second current are not changed.

When current controller 40 controls the first current and the second current as described above, it is possible to supply chloride ions in a necessary amount to first aqueous solution L1 in electrolysis tank 10, and maintain the chloride ion concentration of first aqueous solution L1 at a predetermined concentration.

In the "1. Case of simultaneously flowing first current and second current", the current ratio between the first current and the second current is changed when the chloride ion concentration of first aqueous solution L1 is increased or decreased. Since the first current and the second current simultaneously flow, it is possible to minimize the increase or decrease in the chloride ion concentration of first aqueous solution L1 and maintain the optimum predetermined concentration thereof.

In the "2. Case of flowing first current at predetermined value and simultaneously controlling second current", the second current mainly flows or stops when the chloride ion concentration of first aqueous solution L1 is increased or decreased. Therefore, the chloride ion concentration of first aqueous solution L1 can be maintained at a predetermined concentration.

In the "3. Case of flowing first current or second current", one of the first current and the second current flows, and the other is stopped when the chloride ion concentration of first aqueous solution L1 is increased or decreased. Therefore, the chloride ion concentration of first aqueous solution L1 can be maintained at a predetermined concentration.

In the cases 2 and 3, one of the first current and the second current is controlled, and therefore the current can be easily controlled.

As described above, it is possible to provide space purification device 1 capable of stably generating a desired amount of hypochlorous acid gas because chloride ions consumed in first aqueous solution L1 can be appropriately supplied from second aqueous solution L2. Therefore, it is possible to provide space purification device 1 capable of stably generating a desired amount of hypochlorous acid gas without externally supplying an aqueous solution containing chloride ions for a long period of time, for example, for one year.

Note that a plurality of current controllers 40 may be provided to separately control the first current and the second current.

In Fig. 1, inflow port 15 and outflow port 17 are disposed on the right of the back side (on the positive side of the y-axis and on the positive side of the x-axis) and on the left of this side (on the negative side of the y-axis and on the negative side of the x-axis) when electrolysis tank 10 is viewed in plane, but the disposition is not limited thereto. For example, the positions of inflow port 15 and outflow port 17 may be reversed, inflow port 15 and outflow port 17 may be at the same position on the x-axis, or inflow port 15 and outflow port 17 may be at the same position on the y-axis. However, it is preferable that inflow port 15 and outflow port 17 are disposed at the farthest position in the xy plane. With the disposition, the air flowing thereinto and hypochlorous acid are mixed in mixing space 16 for a longer time, so that a larger amount of hypochlorous acid can be contained in the mixed air.

In addition, a case will be described in which the rectangular plane P1 on the side of anion exchange membrane 30 (yz plane on the positive side of the x-axis) of the electrolysis tank-side anode plate having a plate shape and the rectangular plane P2 on the side of anion exchange membrane 30 (yz plane on the negative side of the x-axis) of the supply tank-side cathode plate having a plate shape do not face each other. When the planes of the rectangles are disposed parallel to the xy plane, a non-uniform electric field may be generated between the electrolysis tank-side anode plate and the supply tank-side cathode plate. When a non-uniform electric field is generated between the electrolysis tank-side anode plate and the supply tank-side cathode plate, the current distribution also becomes non-uniform between the electrolysis tank-side anode plate and the supply tank-side cathode plate.

When the current distribution becomes non-uniform between the electrolysis tank-side anode plate and the supply tank-side cathode plate, there is a high current density region and a low current density region. In the high current density portion, the electrolysis tank-side anode plate and the supply tank-side cathode plate (hereinafter, also referred to as each electrode plate) easily undergo the deterioration of the catalyst layer on their surface; and in the low current density portion, each electrode plate hardly undergoes the deterioration of the catalyst layer on their surface. That is, when a non-uniform electric field is generated between the electrolysis tank-side anode plate and the supply tank-side cathode plate, the current distribution becomes non-uniform, and there can be regions different in current density on the same electrode plate at the same time. Therefore, when each electrode plate is used, the catalyst layer may non-uniformly deteriorate. When the deterioration degree of the catalyst layer differs in the surface of each electrode plate, there can be a region that can be used as an electrode, and a region that cannot be used as a result of deterioration on each electrode plate at the same time, at a certain point during repeated electrolysis. When electrolysis is performed on each electrode plate including a region that cannot be used as an electrode, the electrolysis efficiency may be easily reduced.

On the other hand, in space purification device 1 according to the exemplary embodiment, plane P1 and plane P2 are disposed to face each other via anion exchange membrane 30. With the disposition, an electric field can be uniformly generated between the electrolysis tank-side anode plate and the supply tank-side cathode plate, and therefore the current also uniformly distributes between both electrodes. Therefore, since the catalyst layer uniformly deteriorates in the surface of each electrode plate, it is possible to inhibit non-uniform deterioration of the catalyst layer on the surface of each electrode plate due to a non-uniform electric field even when electrolysis is repeatedly performed. Therefore, it is possible to inhibit the reduction of electrolysis efficiency.

<First modification>

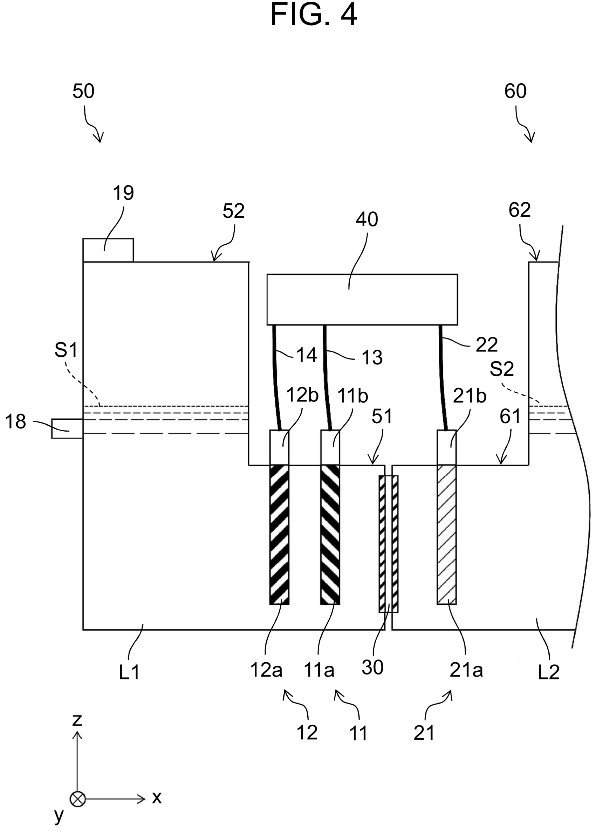

Hereinafter, the first modification of electrolysis tank 10 and supply tank 20 according to the first exemplary embodiment will be described with reference to Fig. 4. Fig. 4 is a front cross-sectional view illustrating the first modification of electrolysis tank 10 and supply tank 20 included in space purification device 1 according to the first exemplary embodiment. The same configurations as those of electrolysis tank 10 and supply tank 20 are denoted by the same reference marks, and the description thereof will be omitted.

First, electrolysis tank 50 as the first modification of electrolysis tank 10 will be described. As illustrated in Fig. 4, electrolysis tank 50 has a substantially L-shape in a front view. Electrolysis tank 50 includes first ceiling surface 51 and second ceiling surface 52, which are two ceiling surfaces different in height (position in z-axis direction). First ceiling surface 51 has a height (position in z-axis direction) lower than that of second ceiling surface 52. The electrolysis tank-side anode plate and the electrolysis tank-side cathode plate are inserted from the upper side (on the positive side of z-axis) of first ceiling surface 51 in the inside direction of electrolysis tank 50 (in the negative direction of the z-axis).

Electrolysis tank-side anode plate immersion part 11a and electrolysis tank-side cathode plate immersion part 12a inserted into electrolysis tank 50 are disposed inside electrolysis tank 50, and entirely immersed in first aqueous solution L1. In other words, first aqueous solution L1 is stored in electrolysis tank 50 such that electrolysis tank-side anode plate immersion part 11a and electrolysis tank-side cathode plate immersion part 12a are entirely immersed therein. That is, first aqueous solution L1 is stored in electrolysis tank 50 such that liquid level S1 of first aqueous solution L1 is higher than the upper ends (ends on the positive side of z-axis) of electrolysis tank-side anode plate immersion part 11a and electrolysis tank-side cathode plate immersion part 12a.

Electrolysis tank-side anode plate protrusion part 11b and electrolysis tank-side cathode plate protrusion part 12b are disposed outside electrolysis tank 50. Electrolysis tank-side anode plate protrusion part 11b and electrolysis tank-side cathode plate protrusion part 12b are electrically connected to current controller 40 via wiring 13 and wiring 14, respectively.

Here, a case where electrolysis tank 50 illustrated in Fig. 4 includes water level detection unit 18 and water supply unit 19 will be described. Similarly to electrolysis tank 10, water level detection unit 18 is at least disposed above (on the positive side of z-axis) the upper ends (ends on the positive side of z-axis) of electrolysis tank-side anode plate immersion part 11a and electrolysis tank-side cathode plate immersion part 12a. Water supply unit 19 may be, for example, provided above second ceiling surface 52, but is disposed at a position where water can be supplied to electrolysis tank 50.

Subsequently, supply tank 60 as the first modification of supply tank 20 will be described. As illustrated in Fig. 4, supply tank 60 has a shape laterally inverted from that of electrolysis tank 50 in a front view. Supply tank 60 includes first ceiling surface 61 and second ceiling surface 62, which are two ceiling surfaces different in height (position in z-axis direction). First ceiling surface 61 has a height (position in z-axis direction) lower than that of second ceiling surface 62. Supply tank-side cathode plate is inserted from the upper side (in the positive direction of z-axis) of first ceiling surface 61 in the inside direction of supply tank 60 (in the negative direction of the z-axis).

Supply tank-side cathode plate immersion part 21a inserted into supply tank 60 is disposed inside supply tank 60, and entirely immersed in second aqueous solution L2. In other words, second aqueous solution L2 is stored in supply tank 60 such that supply tank-side cathode plate immersion part 21a is entirely immersed therein. That is, second aqueous solution L2 is stored in supply tank 60 such that liquid level S2 of second aqueous solution L2 is higher than the upper end (end on the positive side of z-axis) of supply tank-side cathode plate immersion part 21a.

Supply tank-side cathode plate protrusion part 21b is disposed outside supply tank 60. Supply tank-side cathode plate protrusion part 21b is electrically connected to current controller 40 via wiring 22.

<Second modification>

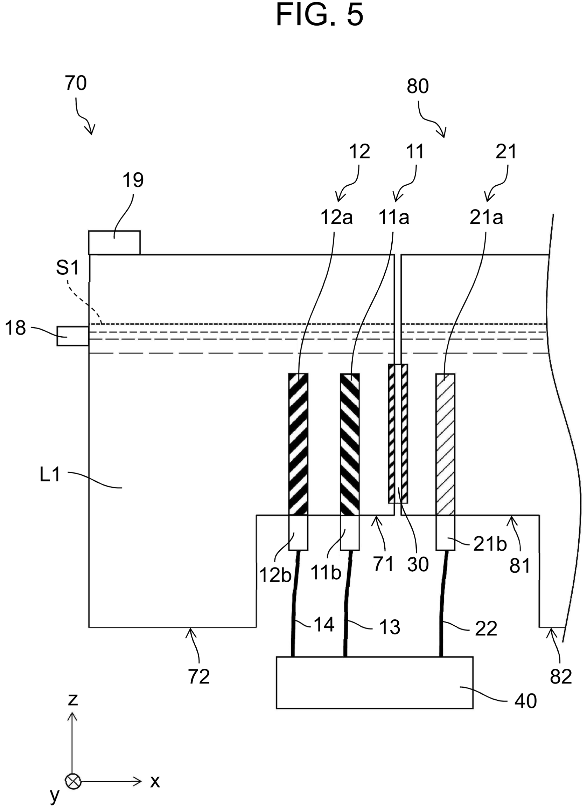

Subsequently, the second modification of electrolysis tank 10 and supply tank 20 according to the first exemplary embodiment will be described with reference to Fig. 5.

Fig. 5 is a front cross-sectional view illustrating the second modification of electrolysis tank 10 and supply tank 20 included in space purification device 1 according to the first exemplary embodiment. The same configurations as those of electrolysis tank 10 and supply tank 20 are denoted by the same reference marks, and the description thereof will be omitted. As illustrated in Fig. 5, electrolysis tank 70 and supply tank 80 have a shape vertically inverted from that of electrolysis tank 50 and supply tank 60 of the first modification.

First, electrolysis tank 70 as the second modification of electrolysis tank 10 will be described. As illustrated in Fig. 5, electrolysis tank 70 has a shape vertically inverted from a substantially L-shape in a front view. Electrolysis tank 70 includes first bottom surface 71 and second bottom surface 72, which are two bottom surfaces different in height (position in z-axis direction). First bottom surface 71 is a bottom surface of electrolysis tank 70 and provided above (in the positive direction of z-axis) second bottom surface 72. The electrolysis tank-side anode plate and the electrolysis tank-side cathode plate are inserted from the lower side (on the negative side of z-axis) of first bottom surface 71 in the inside direction of electrolysis tank 70 (in the positive direction of the z-axis).

Electrolysis tank-side anode plate immersion part 11a and electrolysis tank-side cathode plate immersion part 12a inserted into electrolysis tank 70 are disposed inside electrolysis tank 70, and entirely immersed in first aqueous solution L1. In other words, first aqueous solution L1 is stored in electrolysis tank 70 such that electrolysis tank-side anode plate immersion part 11a and electrolysis tank-side cathode plate immersion part 12a are entirely immersed therein. That is, first aqueous solution L1 is stored in electrolysis tank 70 such that liquid level S1 of first aqueous solution L1 is higher than the upper ends (ends on the positive side of z-axis) of electrolysis tank-side anode plate immersion part 11a and electrolysis tank-side cathode plate immersion part 12a.

Electrolysis tank-side anode plate protrusion part 11b and electrolysis tank-side cathode plate protrusion part 12b are disposed outside electrolysis tank 70. Electrolysis tank-side anode plate protrusion part 11b and electrolysis tank-side cathode plate protrusion part 12b are electrically connected to current controller 40 via wiring 13 and wiring 14, respectively.

In addition, a case where electrolysis tank 70 includes water level detection unit 18 and water supply unit 19 will be described. Similarly to electrolysis tank 10, water level detection unit 18 is at least disposed above (on the positive side of z-axis) the upper ends (ends on the positive side of z-axis) of electrolysis tank-side anode plate immersion part 11a and electrolysis tank-side cathode plate immersion part 12a. Water supply unit 19 is disposed at a position where water can be supplied to electrolysis tank 10, and may be disposed at the upper side of electrolysis tank 70, or may be disposed at the lateral side or the bottom side of electrolysis tank 70.

Subsequently, supply tank 80 as the second modification of supply tank 20 will be described. Supply tank 80 has a shape laterally inverted from that of electrolysis tank 70 in a front view. Supply tank 80 includes first bottom surface 81 and second bottom surface 82, which are two bottom surfaces different in height (position in z-axis direction). First bottom surface 81 is a bottom surface of supply tank 80 and provided above (in the positive direction of z-axis) second bottom surface 82. Supply tank-side cathode plate is inserted from the lower side (on the negative side of z-axis) of first bottom surface 81 in the inside direction of supply tank 80 (in the positive direction of the z-axis).

Supply tank-side cathode plate immersion part 21a inserted into supply tank 80 is disposed inside supply tank 80, and entirely immersed in second aqueous solution L2. In other words, second aqueous solution L2 is stored in supply tank 80 such that supply tank-side cathode plate immersion part 21a is entirely immersed therein. That is, second aqueous solution L2 is stored in supply tank 80 such that liquid level S2 of second aqueous solution L2 is higher than the upper end (end on the positive side of z-axis) of supply tank-side cathode plate immersion part 21a.

Supply tank-side cathode plate protrusion part 21b is disposed outside supply tank 80. Supply tank-side cathode plate protrusion part 21b is electrically connected to current controller 40 via wiring 22.

<Second exemplary embodiment>

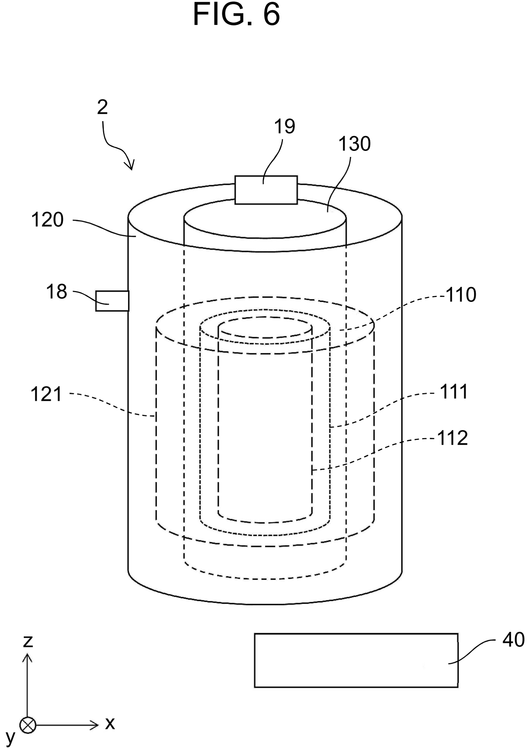

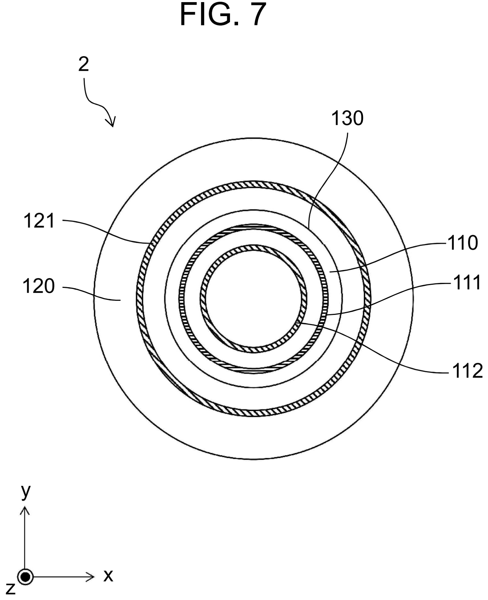

Next, space purification device 2 according to the second exemplary embodiment will be described with reference to Figs. 6 and 7. Note that the same configurations as those in the first exemplary embodiment are denoted by the same reference marks, and the description thereof will be omitted. In addition, although the shape of each configuration is different from those in the first exemplary embodiment, the function of each configuration is the same as those in the first exemplary embodiment, and thus the description of the function of each configuration will be omitted.

Fig. 6 is a front schematic view illustrating space purification device 2 according to the second exemplary embodiment. Fig. 7 is a plan view illustrating space purification device 2 according to the second exemplary embodiment. The hatching shown in Fig. 7 is to clarify each configuration, and Fig. 7 is not a cross-sectional view.

As illustrated in Fig. 6, space purification device 2 according to the exemplary embodiment includes electrolysis tank 110, supply tank 120, anion exchange membrane 130, and current controller 40. Electrolysis tank 110 and supply tank 120 have a tube shape with an open upper surface (the xy plane on the positive side of z-axis) and a bottom surface (the xy plane on the negative side of z-axis). Anion exchange membrane 130 has a tube shape. Anion exchange membrane 130 is disposed between electrolysis tank 110 and supply tank 120. As illustrated in Fig. 6, electrolysis tank 110 is the portion on the inner diameter side of anion exchange membrane 130. The tube shape of electrolysis tank 110, supply tank 120, and anion exchange membrane 130 includes a cylindrical shape and a polygonal tube shape. Although not illustrated in Figs. 6 and 7, first aqueous solution L1 and second aqueous solution L2 are stored in electrolysis tank 110 and supply tank 120, respectively.

As illustrated in Fig. 6, electrolysis tank 110 includes electrolysis tank-side anode 111 and electrolysis tank-side cathode 112. Electrolysis tank-side anode 111 is an electrolysis tank-side anode tube having a tube shape. Electrolysis tank-side cathode 112 is an electrolysis tank-side cathode tube or an electrolysis tank-side cathode rod having a tube shape or a rod shape. Supply tank 120 includes supply tank-side cathode 121. Supply tank-side cathode 121 is a supply tank-side cathode tube having a tube shape. The tube shape of electrolysis tank-side anode 111, electrolysis tank-side cathode 112, and supply tank-side cathode 121 includes a cylindrical shape and a polygonal tube shape. The rod shape includes a cylindrical shape and a spiral shape. Electrolysis tank-side anode 111, electrolysis tank-side cathode 112, and supply tank-side cathode 121 are each electrically connected to current controller 40 via wiring (not shown).

As illustrated in Figs. 6 and 7, space purification device 2 according to the exemplary embodiment has, for example, a nest structure of the cylindrical configurations. More specifically, supply tank 120, supply tank-side cathode 121, anion exchange membrane 130, electrolysis tank 110, electrolysis tank-side anode 111, and electrolysis tank-side cathode 112 are disposed in this order from the outside. That is, the diameters of each of the configurations are as follows: supply tank 120 > supply tank-side cathode 121 > anion exchange membrane 130 = electrolysis tank 110 > electrolysis tank-side anode 111 > electrolysis tank-side cathode 112, and each of the configurations is disposed at predetermined intervals.

The electrolysis tank-side anode tube, and the electrolysis tank-side cathode tube or the electrolysis tank-side cathode rod, are entirely immersed in first aqueous solution L1. The supply tank-side cathode tube is entirely immersed in second aqueous solution L2. As illustrated in Fig. 6, space purification device 2 according to the exemplary embodiment may further include water level detection unit 18 and water supply unit 19. Water level detection unit 18 is disposed above (on the positive side of z-axis) the upper ends (ends on the positive side of z-axis) of electrolysis tank-side anode 111, electrolysis tank-side cathode 112, and supply tank-side cathode 121. Water supply unit 19 is disposed at a position where water can be supplied to electrolysis tank 110, and may be disposed, for example, above (on the positive side of z-axis) electrolysis tank 110.

Electrolysis tank-side anode 111, electrolysis tank-side cathode 112, and supply tank-side cathode 121 may be constituted by a plate member, a mesh member, or an expanded metal.

Space purification device 2 according to the exemplary embodiment includes current controller 40, which is the same as in the first exemplary embodiment. When current controller 40 controls the first current and the second current, it is possible to supply chloride ions in a necessary amount to first aqueous solution L1 in electrolysis tank 110, and maintain the chloride ion concentration of first aqueous solution L1 at a predetermined concentration. Therefore, it is possible to provide space purification device 2 that enables space purification by stably generating a desired amount of hypochlorous acid gas.

Furthermore, space purification device 2 according to the exemplary embodiment can house electrolysis tank 110 and anion exchange membrane 130 on the inner side of supply tank 120. Therefore, it is possible to provide space purification device 2 that is more space-saving and downsized.

Note that the present disclosure is not limited to the above-described exemplary embodiments and modifications, and can be appropriately changed without departing from the gist.

The summary of an embodiment of the present disclosure is as follows.

(Item 1)

Space purification device (1) including:

electrolysis tank (10) configured to store first aqueous solution (L1) containing chloride ions;
supply tank (20) configured to store second aqueous solution (L2) containing chloride ions at a higher concentration than first aqueous solution (L1) and supply chloride ions to first aqueous solution (L1);
electrolysis tank-side anode (11) and electrolysis tank-side cathode (12) each provided in electrolysis tank (10);
supply tank-side cathode (21) provided in supply tank (20);
anion exchange membrane (30) disposed between electrolysis tank (10) and supply tank (20) and capable of permeating anions based on a voltage applied between electrolysis tank-side anode (11) and supply tank-side cathode (21);
non-barrier membrane electrolysis part (E1) provided in electrolysis tank (10) and subjecting first aqueous solution (L1) to non-barrier membrane electrolysis to generate hypochlorous acid with a first current flowing between electrolysis tank-side anode (11) and electrolysis tank-side cathode (12);
barrier membrane electrolysis part (E2) provided over electrolysis tank (10) and supply tank (20) and performing barrier membrane electrolysis via anion exchange membrane (30) with a second current flowing between electrolysis tank-side anode (11) and supply tank-side cathode (21); and
current controller (40) configured to control the second current to supplement chloride ions contained in first aqueous solution (L1) and decreased in number through the non-barrier membrane electrolysis, allowing chloride ions contained in second aqueous solution (L2) to permeate through anion exchange membrane (30) and be supplied to first aqueous solution (L1).

(Item 2)

Space purification device (1) according to item 1, wherein current controller (40) flows the first current and the second current at a predetermined ratio to maintain a chloride ion concentration of first aqueous solution (L1) at a predetermined concentration.

(Item 3)

Space purification device (1) according to item 1 or 2, wherein current controller (40) includes:

voltage acquisition unit (41) configured to acquire a voltage between electrolysis tank-side anode (11) and electrolysis tank-side cathode (12);
calculation unit (42) configured to calculate electric conductivity of first aqueous solution (L1) based on the voltage acquired by voltage acquisition unit (41); and
estimation unit (43) configured to estimate a concentration of first aqueous solution (L1) based on the electric conductivity calculated by calculation unit (42), and
wherein current controller (40):

simultaneously flows the first current and the second current;
changes a current ratio between the first current and the second current to increase an amount of chloride ions permeating through anion exchange membrane (30) from second aqueous solution (L2) and supplied to first aqueous solution (L1), when a chloride ion concentration of first aqueous solution (L1) is lower than a predetermined concentration; and
changes a current ratio between the first current and the second current to decrease an amount of chloride ions permeating through anion exchange membrane (30) from second aqueous solution (L2) and supplied to first aqueous solution (L1), when a chloride ion concentration of first aqueous solution (L1) is higher than the predetermined concentration.

(Item 4)

Space purification device (1) according to item 3, wherein a current ratio between the first current and the second current is not changed when a chloride ion concentration of first aqueous solution (L1) is equal to the predetermined concentration.

(Item 5)

Space purification device (1) according to item 1 or 2, wherein current controller (40) includes:

voltage acquisition unit (41) configured to acquire a voltage between electrolysis tank-side anode (11) and electrolysis tank-side cathode (12);
calculation unit (42) configured to calculate electric conductivity of first aqueous solution (L1) based on the voltage acquired by voltage acquisition unit (41); and
estimation unit (43) configured to estimate a concentration of first aqueous solution (L1) based on the electric conductivity calculated by calculation unit (42), and
wherein current controller (40):

flows the first current at a predetermined value and simultaneously controls the second current; and
when the second current is controlled,
flows the second current to increase an amount of chloride ions permeating through anion exchange membrane (30) from second aqueous solution (L2) and supplied to first aqueous solution (L1), when a chloride ion concentration of first aqueous solution (L1) is lower than a predetermined concentration; and
stops the second current to stop supplying chloride ions from second aqueous solution (L2) to first aqueous solution (L1), when a chloride ion concentration of first aqueous solution (L1) is higher than the predetermined concentration.

(Item 6)

Space purification device (1) according to item 1 or 2, wherein current controller (40) includes:

voltage acquisition unit (41) configured to acquire a voltage between electrolysis tank-side anode (11) and electrolysis tank-side cathode (12);
calculation unit (42) configured to calculate electric conductivity of first aqueous solution (L1) based on the voltage acquired by voltage acquisition unit (41); and
estimation unit (43) configured to estimate a concentration of first aqueous solution (L1) based on the electric conductivity calculated by calculation unit (42), and
wherein current controller (40):

stops the first current and simultaneously flows the second current to increase an amount of chloride ions permeating through anion exchange membrane (30) from second aqueous solution (L2) and supplied to first aqueous solution (L1), when a chloride ion concentration of first aqueous solution (L1) is lower than a predetermined concentration; and
stops the second current and simultaneously flows the first current to stop supplying chloride ions from second aqueous solution (L2) to first aqueous solution (L1), when a chloride ion concentration of first aqueous solution (L1) is higher than the predetermined concentration.

(Item 7)

Space purification device (1) according to any one of items 1 to 6, wherein

electrolysis tank-side anode (11), electrolysis tank-side cathode (12), and supply tank-side cathode (21) are each an electrolysis tank-side anode plate, an electrolysis tank-side cathode plate, and a supply tank-side cathode plate, each having a plate shape;
the electrolysis tank-side anode plate and the electrolysis tank-side cathode plate are inserted to an inside of electrolysis tank (10) from an outside of electrolysis tank (10), and the supply tank-side cathode plate is inserted to an inside of supply tank (20) from an outside of supply tank (20);
the electrolysis tank-side anode plate has electrolysis tank-side anode plate immersion part (11a) disposed inside electrolysis tank (10), and electrolysis tank-side anode plate protrusion part (11b) disposed outside electrolysis tank (10);
the electrolysis tank-side cathode plate has electrolysis tank-side cathode plate immersion part (12a) disposed inside electrolysis tank (10), and electrolysis tank-side cathode plate protrusion part (12b) disposed outside electrolysis tank (10);
the supply tank-side cathode plate has supply tank-side cathode plate immersion part (21a) disposed inside supply tank (20), and supply tank-side cathode plate protrusion part (21b) disposed outside supply tank (20);
electrolysis tank-side anode plate immersion part (11a) and electrolysis tank-side cathode plate immersion part (12a) are entirely immersed in first aqueous solution (L1); and
supply tank-side cathode plate immersion part (21a) is entirely immersed in second aqueous solution (L2).

(Item 8)

Space purification device (1) according to item 7, wherein the electrolysis tank-side anode plate and the electrolysis tank-side cathode plate are inserted to an inside of electrolysis tank (10) from a lateral side of electrolysis tank (10), and the supply tank-side cathode plate is inserted to an inside of supply tank (20) from a lateral side of supply tank (20).

(Item 9)

Space purification device (1) according to item 7, wherein the electrolysis tank-side anode plate and the electrolysis tank-side cathode plate are inserted to an inside of electrolysis tank (10) from an upper side of electrolysis tank (10), and the supply tank-side cathode plate is inserted to an inside of supply tank (20) from an upper side of supply tank (20).

(Item 10)

Space purification device (1) according to item 7, wherein the electrolysis tank-side anode plate and the electrolysis tank-side cathode plate are inserted to an inside of electrolysis tank (10) from a lower side of electrolysis tank (10), and the supply tank-side cathode plate is inserted to an inside of supply tank (20) from a lower side of supply tank (20).

(Item 11)

Space purification device (2) according to any one of items 1 to 6, wherein

electrolysis tank-side anode (111) and supply tank-side cathode (121) are each an electrolysis tank-side anode tube and a supply tank-side cathode tube each having a cylindrical shape or a polygonal tube shape;
electrolysis tank-side cathode (121) is an electrolysis tank-side cathode tube having a cylindrical shape or a polygonal tube shape, or an electrolysis tank-side cathode rod having a rod shape;
the electrolysis tank-side anode tube, and the electrolysis tank-side cathode tube or the electrolysis tank-side cathode rod, are entirely immersed in first aqueous solution (L1); and
the supply tank-side cathode tube is entirely immersed in second aqueous solution (L2).

(Item 12)

Space purification device (2) according to item 11, wherein electrolysis tank (110) and anion exchange membrane (130) are housed on an inner side of supply tank (120).

(Item 13)

Space purification device (1) according to item 7, including:

water level detection unit (18) configured to detect a position of a liquid level of first aqueous solution (L1); and
water supply unit (19) configured to supply water to electrolysis tank (10) to keep the position of the liquid level detected by water level detection unit (18) not lower than upper ends of electrolysis tank-side anode plate immersion part (11a) and electrolysis tank-side cathode plate immersion part (12a).

(Item 14)

Space purification device (2) according to item 11 or 12, further including:

water level detection unit (18) configured to detect a position of a liquid level of first aqueous solution (L1); and
water supply unit (19) configured to supply water to electrolysis tank (110) to keep the position of the liquid level detected by water level detection unit (18) not lower than upper ends of the electrolysis tank-side anode tube, and the electrolysis tank-side cathode tube or the electrolysis tank-side cathode rod.

(Item 15)

Space purification device (1) according to any one of items 7 to 10, wherein
the electrolysis tank-side anode plate and the supply tank-side cathode plate are:

each close to anion exchange membrane (30), and
disposed, planes of the plate shapes facing each other via anion exchange membrane (30); and
the electrolysis tank-side cathode plate is:
disposed, a plane of the plate shape facing the plane of the plate shape of the electrolysis tank-side anode plate.

(Item 16)

Space purification device (1) according to any one of items 7 to 10, wherein for each of the electrolysis tank-side anode plate, the electrolysis tank-side cathode plate, and the supply tank-side cathode plate,

the plate shape is a rectangle;

a short direction of the rectangle is disposed along a vertical direction; and
a long direction of the rectangle is disposed along a horizontal direction.

(Item 17)

Space purification device (1,2) according to any one of items 1 to 16, wherein non-barrier membrane electrolysis part (E1) includes:

electrolysis tank-side anode (11,111), and
electrolysis tank-side cathode (12,112); and
barrier membrane electrolysis part (E2) includes:

electrolysis tank-side anode (11,111),
supply tank-side cathode (21,121), and
anion exchange membrane (30,130).

(Item 18)

Space purification device (1,2) according to any one of items 1 to 10, including:

case (B) housing electrolysis tank (10) and supply tank (20);
inflow port (15) disposed above a liquid level of first aqueous solution (L1) stored in electrolysis tank (10) inside case (B), air from an outside space of case (B) flowing into inflow port (15);
mixing space (16) mixing the volatilized hypochlorous acid and the air having flowed in from inflow port (15); and
outflow port (17) flowing out a mixed air mixed in the mixing space to the outside space.

(Item 19)

Space purification device (1) according to any one of items 1 to 10, wherein each surface where electrolysis tank (10) and supply tank (20) face each other is formed of a frame member, and
anion exchange membrane (30) is fitted into the frame members and anion exchange membrane (30) is disposed between electrolysis tank (10) and supply tank (20).

REFERENCE MARKS IN THE DRAWINGS

| 1, 2 | space purification device |
|---|---|
| 10, 50, 70, 110 | electrolysis tank |
| 11, 111 | electrolysis tank-side anode |
| 11a | electrolysis tank-side anode plate immersion part |
| 11b | electrolysis tank-side anode plate protrusion part |
| 12, 112 | electrolysis tank-side cathode |
| 12a | electrolysis tank-side cathode plate immersion part |
| 12b | electrolysis tank-side cathode plate protrusion part |
| 15 | inflow port |
| 16 | mixing space |
| 17 | outflow port |
| 18 | water level detection unit |
| 19 | water supply unit |
| 20, 60, 80, 120 | supply tank |
| 21, 121 | supply tank-side cathode |
| 21a | supply tank-side cathode plate immersion part |
| 21b | supply tank-side cathode plate protrusion part |
| 30, 130 | anion exchange membrane |
| 40 | current controller |
| 41 | voltage acquisition unit |
| 42 | calculation unit |

| 43 | estimation unit |
|---|---|
| B | case |
| E1 | non-barrier membrane electrolysis part |
| E2 | barrier membrane electrolysis part |
| L1 | first aqueous solution |
| L2 | second aqueous solution |

**Claims**

1. A space purification device comprising:

   an electrolysis tank configured to store a first aqueous solution containing chloride ions;
   a supply tank configured to store a second aqueous solution containing chloride ions at a higher concentration than the first aqueous solution and supply chloride ions to the first aqueous solution;
   an electrolysis tank-side anode and an electrolysis tank-side cathode each provided in the electrolysis tank;
   a supply tank-side cathode provided in the supply tank;
   an anion exchange membrane disposed between the electrolysis tank and the supply tank and capable of permeating anions based on a voltage applied between the electrolysis tank-side anode and the supply tank-side cathode;
   a non-barrier membrane electrolysis part provided in the electrolysis tank and subjecting the first aqueous solution to non-barrier membrane electrolysis to generate hypochlorous acid with a first current flowing between the electrolysis tank-side anode and the electrolysis tank-side cathode;
   a barrier membrane electrolysis part provided over the electrolysis tank and the supply tank and performing barrier membrane electrolysis via the anion exchange membrane with a second current flowing between the electrolysis tank-side anode and the supply tank-side cathode; and
   a current controller configured to control the second current to supplement chloride ions contained in the first aqueous solution and decreased in number through the non-barrier membrane electrolysis, allowing chloride ions contained in the second aqueous solution to permeate through the anion exchange membrane and be supplied to the first aqueous solution.

2. The space purification device according to claim 1, wherein the current controller flows the first current and the second current at a predetermined ratio to maintain a chloride ion concentration of the first aqueous solution at a predetermined concentration.

3. The space purification device according to claim 1, wherein the current controller includes:

   a voltage acquisition unit configured to acquire a voltage between the electrolysis tank-side anode and the electrolysis tank-side cathode;
   a calculation unit configured to calculate electric conductivity of the first aqueous solution based on the voltage acquired by the voltage acquisition unit; and
   an estimation unit configured to estimate a concentration of the first aqueous solution based on the electric conductivity calculated by the calculation unit, and
   wherein the current controller:

      simultaneously flows the first current and the second current;
      changes a current ratio between the first current and the second current to increase an amount of chloride ions permeating through the anion exchange membrane from the second aqueous solution and supplied to the first aqueous solution, when a chloride ion concentration of the first aqueous solution is lower than a predetermined concentration; and
      changes a current ratio between the first current and the second current to decrease an amount of chloride ions permeating through the anion exchange membrane from the second aqueous solution and supplied to the first aqueous solution, when a chloride ion concentration of the first aqueous solution is higher than the predetermined concentration.

4. The space purification device according to claim 3, wherein a current ratio between the first current and the second current is not changed when a chloride ion concentration of the first aqueous solution is equal to the predetermined concentration.

**5.** The space purification device according to claim 1, wherein the current controller includes:

a voltage acquisition unit configured to acquire a voltage between the electrolysis tank-side anode and the electrolysis tank-side cathode;

a calculation unit configured to calculate electric conductivity of the first aqueous solution based on the voltage acquired by the voltage acquisition unit; and

an estimation unit configured to estimate a concentration of the first aqueous solution based on the electric conductivity calculated by the calculation unit, and

wherein the current controller:

flows the first current at a predetermined value and simultaneously controls the second current; and when the second current is controlled,

flows the second current to increase an amount of chloride ions permeating through the anion exchange membrane from the second aqueous solution and supplied to the first aqueous solution, when a chloride ion concentration of the first aqueous solution is lower than a predetermined concentration; and

stops the second current to stop supplying chloride ions from the second aqueous solution to the first aqueous solution, when a chloride ion concentration of the first aqueous solution is higher than the predetermined concentration.

**6.** The space purification device according to claim 1, wherein the current controller includes:

a voltage acquisition unit configured to acquire a voltage between the electrolysis tank-side anode and the electrolysis tank-side cathode;

a calculation unit configured to calculate electric conductivity of the first aqueous solution based on the voltage acquired by the voltage acquisition unit; and

an estimation unit configured to estimate a concentration of the first aqueous solution based on the electric conductivity calculated by the calculation unit, and

wherein the current controller:

stops the first current and simultaneously flows the second current to increase an amount of chloride ions permeating through the anion exchange membrane from the second aqueous solution and supplied to the first aqueous solution, when a chloride ion concentration of the first aqueous solution is lower than a predetermined concentration; and

stops the second current and simultaneously flows the first current to stop supplying chloride ions from the second aqueous solution to the first aqueous solution, when a chloride ion concentration of the first aqueous solution is higher than the predetermined concentration.

**7.** The space purification device according to claim 1, wherein

the electrolysis tank-side anode, the electrolysis tank-side cathode, and the supply tank-side cathode are each an electrolysis tank-side anode plate, an electrolysis tank-side cathode plate, and a supply tank-side cathode plate, each having a plate shape;

the electrolysis tank-side anode plate and the electrolysis tank-side cathode plate are inserted to an inside of the electrolysis tank from an outside of the electrolysis tank, and the supply tank-side cathode plate is inserted to an inside of the supply tank from an outside of the supply tank;

the electrolysis tank-side anode plate has an electrolysis tank-side anode plate immersion part disposed inside the electrolysis tank, and an electrolysis tank-side anode plate protrusion part disposed outside the electrolysis tank;

the electrolysis tank-side cathode plate has an electrolysis tank-side cathode plate immersion part disposed inside the electrolysis tank, and an electrolysis tank-side cathode plate protrusion part disposed outside the electrolysis tank;

the supply tank-side cathode plate has a supply tank-side cathode plate immersion part disposed inside the supply tank, and a supply tank-side cathode plate protrusion part disposed outside the supply tank;

the electrolysis tank-side anode plate immersion part and the electrolysis tank-side cathode plate immersion part are entirely immersed in the first aqueous solution; and

the supply tank-side cathode plate immersion part is entirely immersed in the second aqueous solution.

**8.** The space purification device according to claim 1, wherein

the electrolysis tank-side anode and the supply tank-side cathode are each an electrolysis tank-side anode tube and a supply tank-side cathode tube each having a cylindrical shape or a polygonal tube shape;

the electrolysis tank-side cathode is an electrolysis tank-side cathode tube having a cylindrical shape or a polygonal tube shape, or an electrolysis tank-side cathode rod having a rod shape;

the electrolysis tank-side anode tube, and the electrolysis tank-side cathode tube or the electrolysis tank-side cathode rod, are entirely immersed in the first aqueous solution; and

the supply tank-side cathode tube is entirely immersed in the second aqueous solution.

9. The space purification device according to claim 7, comprising:

a water level detection unit configured to detect a position of a liquid level of the first aqueous solution; and a water supply unit configured to supply water to the electrolysis tank to keep the position of the liquid level detected by the water level detection unit not lower than upper ends of the electrolysis tank-side anode plate immersion part and the electrolysis tank-side cathode plate immersion part.

10. The space purification device according to claim 7, wherein the electrolysis tank-side anode plate and the supply tank-side cathode plate are:

each close to the anion exchange membrane, and disposed, planes of the plate shapes facing each other via the anion exchange membrane; and the electrolysis tank-side cathode plate is:
disposed, a plane of the plate shape facing the plane of the plate shape of the electrolysis tank-side anode plate.

11. The space purification device according to claim 7, wherein for each of the electrolysis tank-side anode plate, the electrolysis tank-side cathode plate, and the supply tank-side cathode plate,

the plate shape is a rectangle;
a short direction of the rectangle is disposed along a vertical direction; and
a long direction of the rectangle is disposed along a horizontal direction.

12. The space purification device according to claim 1, wherein the non-barrier membrane electrolysis part includes:

the electrolysis tank-side anode, and
the electrolysis tank-side cathode; and
the barrier membrane electrolysis part includes:

the electrolysis tank-side anode,
the supply tank-side cathode, and
the anion exchange membrane.

13. The space purification device according to claim 1, comprising:

a case housing the electrolysis tank and the supply tank;
an inflow port disposed above a liquid level of the first aqueous solution stored in the electrolysis tank inside the case, air from an outside space of the case flowing into the inflow port;
a mixing space mixing the volatilized hypochlorous acid and the air having flowed in from the inflow port; and
an outflow port flowing out a mixed air mixed in the mixing space to the outside space.

# FIG. 1

# FIG. 2

# FIG. 3

```
┌─────────────────────────────────────┐ ⌐ 40
│                                      │
│  ┌────────────────────────────┐  ⌐ 41
│  │  VOLTAGE ACQUISITION       │      │
│  │  UNIT                      │      │
│  └────────────────────────────┘      │
│                                      │
│                                      │
│  ┌────────────────────────────┐  ⌐ 42
│  │  CALCULATION UNIT          │      │
│  └────────────────────────────┘      │
│                                      │
│                                      │
│  ┌────────────────────────────┐  ⌐ 43
│  │  ESTIMATION UNIT           │      │
│  └────────────────────────────┘      │
│                                      │
└──────────────────────────────────────┘
```

# FIG. 4

# FIG. 5

# FIG. 6

# FIG. 7

## INTERNATIONAL SEARCH REPORT

International application No.

**PCT/JP2024/020195**

| A. | CLASSIFICATION OF SUBJECT MATTER |
|---|---|

*A61L 9/01*(2006.01)i; *C02F 1/461*(2023.01)i; *F24F 8/24*(2021.01)i
FI: A61L9/01 F; C02F1/461 Z; F24F8/24

According to International Patent Classification (IPC) or to both national classification and IPC

| B. | FIELDS SEARCHED |
|---|---|

Minimum documentation searched (classification system followed by classification symbols)

A61L9/01; C02F1/461; F24F8/24

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2016-168542 A (KABUSHIKI KAISHA TOSHIBA) 23 September 2016 (2016-09-23) claims, paragraphs [0004], [0025], [0033], [0037], [0085] | 1, 7-8, 10-13 |
| Y | | 2-6, 9 |
| Y | JP 2022-182221 A (PANASONIC INTELLECTUAL PROPERTY MANAGEMENT CO., LTD.) 08 December 2022 (2022-12-08) paragraphs [0001], [0006]-[0010], [0025]-[0035] | 2-6, 9 |

☐ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 August 2024** | **20 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/020195**

| Patent document cited in search report | | | Publication date (day/month/year) | Patent family member(s) | Publication date (day/month/year) |
|---|---|---|---|---|---|
| JP | 2016-168542 | A | 23 September 2016 | (Family: none) | |
| JP | 2022-182221 | A | 08 December 2022 | (Family: none) | |

Form PCT/ISA/210 (patent family annex) (July 2022)

## REFERENCES CITED IN THE DESCRIPTION

**Patent documents cited in the description**

• JP 2019174032 A